(19) Europäisches Patentamt
European Patent Office
Office européen des brevets

(11) Veröffentlichungsnummer: **0 117 550 B1**

(12) **EUROPÄISCHE PATENTSCHRIFT**

(45) Veröffentlichungstag der Patentschrift: **08.05.91**

(51) Int. Cl.5: **C12N 9/02**, C12Q 1/26, //(C12N9/02,C12R1:01,1:225, 1:46)

(21) Anmeldenummer: **84101954.0**

(22) Anmeldetag: **24.02.84**

Die Akte enthält technische Angaben, die nach dem Eingang der Anmeldung eingereicht wurden und die nicht in dieser Patentschrift enthalten sind.

# (54) Pyruvatoxidase.

(30) Priorität: **25.02.83 DE 3306719**

(43) Veröffentlichungstag der Anmeldung: **05.09.84 Patentblatt 84/36**

(45) Bekanntmachung des Hinweises auf die Patenterteilung: **08.05.91 Patentblatt 91/19**

(84) Benannte Vertragsstaaten: **AT BE CH DE FR GB IT LI LU NL SE**

(56) Entgegenhaltungen:
**FR-A- 2 436 182**

**CHEMICAL ABSTRACTS, Band 52, Nr. 22, 25. November 1958, Spalte 20374h, Columbus, Ohio, US; KEI YAMANAKA: "Pyruvate metabolism by lactobacilli. I. Pyruvic oxidase"**

**CHEMICAL ABSTRACTS, Band 52, Nr. 22, 25. November 1958, Spalte 20375b, Columbus, Ohio, US; KEI YAMANAKA: "Pyruvate metabolism by lactic acid bacteria. I. Aerobic metabolism of pyruvate"**

(73) Patentinhaber: **BOEHRINGER MANNHEIM GMBH
Patentabteilung, Abt. E Sandhofer Strasse 112-132 Postfach 31 01 20
W-6800 Mannheim 31 Waldhof(DE)**

(72) Erfinder: **Elstner, Erich, Prof. Dr.
Wildmoosstrasse 18
W-8030 Gröbenzell(DE)**
Erfinder: **Schleifer, Karl-Heinz, Prof. Dr.
Schwalbenstrasse 3a
W-8044 Unterschleissheim(DE)**
Erfinder: **Götz, Friedrich, Dr.
Pferseer Strasse 15
W-8900 Augsburg(DE)**
Erfinder: **Sedewitz, Barbara
Türkenstrasse 82
W-8000 München 40(DE)**
Erfinder: **Röder, Albert, Dr.
Tiefentalweg 8
W-8124 Seeshaupt(DE)**

**EP 0 117 550 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 57, Nr. 13, 24. Dezember 1962, Spalte 17176f, Columbus, Ohio, US; V. SCARDOVI: "Oxidative activity in Pediococcus. I. Metabolism of pyruvic acid and characteristic of Lactobacillus delbrueckii. II. Oxidation of reduced diphosphopyridine nucleotide in Pediococcus casei", & ANN. MICROBIOL. ENZIMOL. 1962, 12 (1-3), 1-32

CHEMICAL ABSTRACTS, Band 92, Nr. 25, 23. Juni 1980, Seite 289, Nr. 211562x, Columbus, Ohio, US; F. GOETZ et al.: "Oxygen utilization by Lactobacillus plantarum. I. Oxygen consuming reactions", & ARCH. MICROBIOL. 1980, 125(3), 209-14

CHEMICAL ABSTRACTS, Band 102, Nr. 3, Januar 1985, Seite 546, Nr. 22815w, Columbus, Ohio, US

Erfinder: Möllering, Hans
Herrestrasse 10
W-8132 Tutzing(DE)
Erfinder: Seidel, Hans, Dr.
Waxensteinstrasse 6
W-8132 Tutzing(DE)

(74) Vertreter: Weickmann, Heinrich, Dipl.-Ing. et al
Patentanwälte Dipl.-Ing. H.Weickmann
Dipl.-Phys.Dr. K.Fincke Dipl.-Ing.
F.A.Weickmann Dipl.-Chem. B. Huber Dr.-Ing.
H. Liska Dipl.-Phys.Dr. J. Prechtel Postfach 860820
W-8000 München 86(DE)

## Beschreibung

Die Erfindung betrifft eine neue Pyruvatoxidase, ein Verfahren zu ihrer Herstellung und ihre Verwendung.

Pyruvatoxidase E.C. 1.2.3.3 ist ein Enzym, welches Pyruvat in Gegenwart von Phosphationen und Sauerstoff decarboxyliert unter Bildung von $H_2O_2$ (Federation Proceedings 13 (1954) 734-738). Von den Reaktionsprodukten Acetylphosphat, $CO_2$ und $H_2O_2$ ist insbesondere letzteres analytisch gut erfaßbar und daher eignet sich dieses Enzym für die quantitative Bestimmung von Pyruvat und von Pyruvat bildenden Enzymen bzw. deren Substraten.

Eine charakteristische Eigenschaft der bekannten Pyruvatoxidase besteht darin, daß sie zur Aktivität einen Zusatz von FAD (Flavin-Adenin-Dinukleotid), TPP (Thiaminpyrophosphat) und von zweiwertigen Metallionen benötigt. Diese Eigenschaft ist jedoch im Rahmen eines Verfahrens bzw. Reagenz zur Bestimmung von Pyruvat oder Pyruvat bildenden Reaktionen störend. Insbesondere TPP neigt nämlich in Gegenwart von Serum und von Magnesiumionen zur Bildung von unlöslichen Niederschlägen, die Trübungen verursachen und daher die optische Messbarkeit beeinträchtigen. Ferner ist die Lagerungsfähigkeit von TPP in einem kombinierten Reagenz, insbesondere in Gegenwart von zweiwertigen Metallionen, verringert, so daß es sich bei der Lagerung allmählich zersetzt. Letztere Eigenschaft ist insbesondere störend im Rahmen eines kinetischen Naßtests. Daher wäre es sehr erwünscht, für die Bestimmung von Pyruvat und ein hierfür geeignetes Reagenz über eine Pyruvatoxidase zu verfügen, welche von den genannten Substanzen, insbesondere aber von TPP und zweiwertigen Metallionen unabhängig ist, d. h. auch ohne Zusatz derselben eine ausreichend hohe Aktivität aufweist.

Überraschenderweise wurde nunmehr gefunden und hierauf basiert die Erfindung, daß eine Pyruvatoxidase existiert, welche ohne Zusatz von FAD, TPP und zweiwertigen Metallionen aktiv ist.

Gegenstand der Erfindung ist daher eine Pyruvatoxidase, die dadurch gekennzeichnet ist, daß sie ohne Zusatz von FAD, TPP und zweiwertigen Metallionen Pyruvat unter Bildung von $H_2O_2$ decarboxyliert, erhältlich aus Lactobacillus plantarum, DSM 2571, Lactobacillus salivarius, DSM 2573, Leuconostoc mesenteroides, DSM 2572, oder Streptococcus cremoris, DSM 2574.

Die erfindungsgemäße Pyruvatoxidase unterscheidet sich aber auch durch ihre sonstigen Eigenschaften teilweise erheblich von der bekannten Pyruvatoxidase, wie sie beispielsweise in der DE-OS 29 11 481 beschrieben ist. Insbesondere liegt der optimale pH-Bereich für die Aktivität beim erfindungsgemäßen Enzym tiefer (pH 5,0 bis 6,5, optimal 5,7) als beim bekannten Enzym (pH 6,5 bis 7,5), das Molekulargewicht liegt beim erfindungsgemäßen Enzym niedriger (146.000), bestimmt in der Ultrazentrifuge, als beim bekannten Enzym (190.000), bestimmt mittels Gelfiltration Sephadex G 200, und, was für die Verwendung zur Pyruvatbestimmung besonders wichtig ist, die Michaelis Konstante $K_M$ für Pyruvat ist um mehr als eine Zehnerpotenz niedriger. Im folgenden werden Eigenschaften des erfindungsgemäßen Enzyms mit Eigenschaften des bekannten Enzyms verglichen:

| Eigenschaft | Erfindung | Pyruvat-Oxidase gemäß DE-OS 2911481 |
|---|---|---|
| $K_M$ Pyruvat (25 °C) | 0,4 mmol/l | 10,2 mmol/l |
| $K_M$ Phosphat (25 °C) | 2,3 mmol/l | 0,96 mmol/l |
| Stabilitätsoptimum | pH 5,6 bis 5,8 | pH 6,5 bis 7,5 |

Substratspezifität:

| | Erfindung | Pyruvatoxidase gemäß DE-OS 29 11 481 |
|---|---|---|
| Pyruvat | 100 % | 100 % |
| α-Ketobutyrat | 0 % | 3 % |
| Acetaldehyd | 15 % | 0 % |
| Methylglyoxal | 20 % | 0 % |

Den Einfluß von Inhibitoren im Vergleich zum bekannten Enzym zeigt nachstehende Tabelle 1.

<u>Tabelle 1</u>

| Inhibitor | Konzentration in mmol/l | Pyruvat-Oxidase gemäß DE-OS 2911481 (Aktivität in %) | Pyruvat-Oxidase gemäß Erfindung (Aktivität in %) |
|---|---|---|---|
| ohne | 0 | 100 | 100 |
| KCN | 10 | 85 | 75 |
| | 100 | 0 | 5 |
| $NaN_3$ | 100 | 75 | 62 |
| EDTA | 0,01 | 78 | 100 |
| | 0,1 | 30 | 100 |
| | 1 | 7 | 98 |
| | 10 | 0 | 85 |

Den Einfluß von Aktivatoren im Vergleich zum bekannten Enzym zeigt nachstehende Tabelle 2.

Tabelle 2

| Aktivator | Konzentration mmol/l | Pyruvat-Oxidase gemäß DE-OS 29 11 481 | Erfindung |
|---|---|---|---|
| | | Aktivität in % | |
| Thiamin-PP + $MnSO_4$ | 0,2<br>1 | 100 | 100 |
| 0 | 0 | 0,2 | 84 |
| $CoCl_2$ | 1 | 0 | 97 |
| $MgSO_4$ | 0,1 | 0,2 | 97 |
| $MnSO_4$ | 0,1 | 13 | 97 |
| Thiamin-PP | 0,2 | 46 | 97 |
| FAD | 0,1 | 0 | 93 |
| FAD + TPP | 0,4+0,2 | 61 | 93 |
| $MgSO_4$ + TPP | 0,4+0,2 | 55 | 100 |

Die obigen Werte zeigen, daß die Hemmwirkung von EDTA auf das erfindungsgemäße Enzym ca. tausendmal schwächer ist als beim bekannten Enzym und nach mehrtägiger Dialyse des erfindungsgemäßen Enzyms durch Zusatz von TPP, FAD und $Mn^{2+}$ die Aktivität nur um 5 bis 15 % gesteigert werden kann, während das bekannte Enzym ohne diese Aktivatoren nicht aktiv ist.

Zwischen dem erfindungsgemäßen Enzym und dem bekannten Enzym besteht im Ouchterlony-Test (Kaninchen) keine immunologische Kreuzreaktion.

Die Gewinnung des erfindungsgemäßen Enzyms erfolgt aus Pyruvatoxidase enthaltenden Mikroorganismen, die auf einem pyruvathaltigen Medium gezüchtet worden sind, unter Anwendung üblicher biochemischer Methoden, wobei für jeden Anreicherungsschritt anhand der charakteristischen Eigenschaften des Enzyms, nämlich $H_2O_2$-Bildung aus Pyruvat in Abwesenheit von FAD, TPP und zweiwertigen Metallionen, leicht nachgewiesen werden kann, in welcher Fraktion sich das gesuchte Enzym befindet. Vorzugsweise versetzt man den wässrigen Mikroorganismenaufschluß direkt oder nach Abtrennung der ungelösten Anteile mit Polyäthylenimin und trennt das hierbei gebildete Unlösliche ab.

Als Quelle für das erfindungsgemaße Enzym verwendet man vorzugsweise Milchsäurebakterien. In Bezug auf ihren Gehalt an dem gewünschten Enzym erwiesen sich Lactobacillus plantarum DSM 2571, Lactobacillus salivarius DSM 2573, Leuconostoc mesenteroides DSM 2572 oder/und Streptococcus cremoris DSM 2574 als besonders geeignete Quellen. Bei Untersuchung von mehreren hundert verschiedenen Milchsäurebakterien wurde das erfindungsgemäße Enzym in etwa einem Viertel der untersuchten Stämme nachgewiesen. Daraus kann geschlossen werden, daß das Enzym weit verbreitet ist. Außerdem liegen Anhaltspunkte dafür vor, daß es auch in Mikroorganismen, die nicht zu den Milchsäurebakterien gehören,

zu finden ist.

Zur Gewinnung des Enzyms aus den Mikroorganismen werden letztere nach üblichen Methoden aufgeschlossen. Gute Ergebnisse wurden mit Ultraschall und in der Dynomühle (Glasperlen) erzielt. Auch der Aufschluß in der Hochdruckdispersion ergab gute Resultate. Allgemein sind aber auch die übrigen Aufschlußmethoden, wie sie beispielsweise in der DE-OS 29 11 481 angegeben sind, anwendbar.

Die Anreicherung und Reinigung des erfindungsgemäßen Enzyms ließ sich, wie bereits erwähnt, nach den üblichen biochemischen Methoden auf verschiedene Weise erzielen. Gemäß einer erprobten Arbeitsweise wird nach dem Aufschluß von Unlöslichem abzentrifugiert, der Überstand zwischen 25 und 65 % Ammoniumsulfat bei pH 6,3 fraktioniert, die aktive Fraktion über Dextranblau-Sepharose (Firma Pharmacia, Upsala, Schweden) chromatographiert, die aktive Fraktion erneut mit Ammoniumsulfat zwischen 20 und 30 % Sättigung fraktioniert und schließlich durch Molekularsiebpassage (Sephadex ACA 34 der Firma LKB) gereinigt.

Bei einer alternativen Methode wurde die Aufschlußflüssigkeit mit 4 % Polyäthylenimin versetzt, der Niederschlag entfernt und im Überstand eine Ammonsulfatfällung bei 65 % Sättigung durchgeführt. Der Niederschlag wird aufgenommen und nach Dialyse einem Saccharosegradientenschritt unterzogen. Nach erneuter Ammonsulfatfällung bis 65 % und Gelfiltration wird über Dextranblau-Sepharose die Feinreinigung vorgenommen.

Durch die beschriebenen Verfahren läßt sich eine etwa 20- bis 30fache Anreicherung erzielen bis auf eine spezifische Aktivität von etwa 6 bis 9 U/mg Protein.

Gemäß einem weiteren Gegenstand der Erfindung verwendet man das neue Enzym zur Bestimmung von Pyruvat durch Messung von gebildetem $H_2O_2$. Hierfür sind zahlreiche geeignete Methoden bekannt, die hier nicht gesondert beschrieben werden müssen. Ebenfalls gut geeignet ist die Messung des $O_2$-Verbrauchs, beispielsweise mittels Sauerstoffelektrode.

Das erfindungsgemäße Enzym kann dabei nicht nur zur Bestimmung des Pyruvats an sich sondern insbesondere auch im Rahmen der Bestimmung von Substraten und Enzymen, die zu einer Pyruvatbildung führen, verwendet werden.

Typische Beispiele für Bestimmungen, die mit dem erfindungsgemäßen Enzym durchgeführt werden können sind:
Glutamat-Pyruvat-Transaminase oder α-Ketoglutarat;
Glutamat-Oxalacetat-Transaminase;
Pyruvatkinase oder ADP;
Lactatdehydrogenase oder Milchsäure;
Glycerin oder Glycerophosphatkinase;
Triglycerid;
Creatinphosphokinase oder Creatin;
Myokinase, Thiokinase oder Fettsäure.

Ein weiterer Gegenstand der Erfindung ist das Reagenz zur Bestimmung von Pyruvat, welches dadurch gekennzeichnet ist, daß es erfindungsgemäße Pyruvatoxidase, Phosphat, Puffer, ein System zur Bestimmung von $H_2O_2$ und gegebenenfalls ein System zur Bildung von Pyruvat enthält.

Als Puffer läßt sich jede geeignete Puffersubstanz verwenden, die in den oben angegebenen pH-Bereichen, in denen das Enzym aktiv und stabil ist, puffert. Gut geeignet ist auch Phosphatpuffer, so daß ein gesonderter Pufferzusatz neben dem für die Reaktion erforderlichen Phosphat nicht mehr nötig ist. Bei der Auswahl des Puffers ist jedoch in der Regel auch zu berücksichtigen, welche pH-Werte für die Hilfsenzyme im System zur Bestimmung von $H_2O_2$ oder im System zur Bildung von Pyruvat enthalten sind. Anhand der bekannten Daten dieser Enzyme kann der Fachmann jedoch ohne weiteres die geeignete Auswahl für den Puffer treffen.

Das erfindungsgemäße Reagenz eignet sich gut zur Imprägnierung von Trägermaterialien wie Papier, Kunststoffolien und dergleichen für Teststreifen.

Vorzugsweise enthält das erfindungsgemäße Reagenz 1 bis 50 U/ml Pyruvatoxidase, 10 bis 500 mmol/l Phosphat, Puffer pH 6 bis 8 (gegebenenfalls 1 bis 50 mmol/l Substrat, z. B. für GPT-Messung je α - Ketoglutarat und l-Alanin) und als System zur Bestimmung von $H_2O_2$ 0,5 bis 40 mmol/l 4-Aminoantipyrin, 1 bis 50 mmol/l 2-Hydroxy-3,5-dichlorbenzol-sulfonsäure (HDBS) und 0,2 bis 20 U/ml Peroxidase.

Durch die Erfindung werden die Nachteile des bekannten Enzymes überwunden und wird die Möglichkeit zur Herstellung eines Reagenzes mit überlegener Lagerungsfähigkeit erzielt.

Die folgenden Beispiele erläutern die Erfindung weiter.

**Beispiel 1**

Isolierung von Pyruvat-Oxidase (Py-OD) aus Lactobacillus plantarum DSM 2571

1. Züchtung

Kultivierungsmedium

pro l: 10 g Bacto-Trypton/Oxoid - 4 g Hefe-Extrakt-Oxoid - 2 g $K_2HPO_4$ x 3 $H_2O$ - 2 g Di-ammoniumhydrogencitrat - 1 ml Tween 80 (Polyoxiethylensorbitanmonooleat) - 0,2 g $MgSO_4$ x 7 $H_2O$ - 0,2 g $MnSO_4$ x $H_2O$ - 0,02 g Molybdänsulfid - 10 g Lactose - 3,2 g Brenztraubensäure pH 7.

150 ml Medium werden von Stichkulturen der Zusammensetzung s.o. oder dem für Milchsäurebakterien üblichen MRS * -Medium beimpft und bei 28 °C geschüttelt. Nach erfolgtem Wachstum werden erneut 150 ml Medium (s.o.) mit 15 % der gewachsenen Kultur beimpft und im 500 ml Erlenmeyer bei 28° C geschüttelt bis zum Abschluß der späten Log-Phase.

2. Isolierung

Aus einer 50 Liter-Kultur Lactobacillus pl. DSM 2571 mit 120 U/1 Py-OD wurden 300 g Feuchtmasse durch Zentrifugation geerntet. 100 g Zellen werden in der Glas-Dynomühle aufgeschlossen, nach Zugabe von 4 % Polyethylenimin zentrifugiert und der Überstand weiter über die Schritte Ammoniumsulfat (25 bis 65 %)-Fraktionierung bei pH 6,3, Dextran-Blau-Sepharose-Chromatographie (Firma Pharmacia), zweite Ammoniumsulfat-Fraktionierung zwischen 20 und 30 % und Molekularsieb-Passage über Sephadex ACA 34 (Firma LKB) aufgearbeitet.

Die Daten der Anreicherung sind in folgender Tabelle zusammengefaßt:

| Schritt | Vol. (ml) | Units | Protein (mg) | Spez.Akt. | Ausbeute % |
|---|---|---|---|---|---|
| Aufschluß-Überst. | 480 | 2516 | 6700 | 0,37 | 100 |
| 1. AS-Fraktionierung | 98 | 2436 | 5540 | 0,44 | 97 |
| Dextranblau-Sepharose | 54 | 2284 | 2560 | 0,89 | 91 |
| 2. AS-Fraktionierung | 13 | 748 | 753 | 1,0 | 30 |
| ACA 34-Mol.-Sieb | 30 | 590 | 102 | 5,8 | 23,5 |

Das gereinigte Enzym hat eine spezifische Aktivität von 5,8 U/mg und enthält weniger als 0,03 % Apo-Glutamat-Pyruvat-Transaminase + α -Ketoglutarat-Oxidase sowie unter 0,002 % NADH-Oxidase.

Die Aktivität der Pyruvat-Oxidase wurde unter folgenden Bedingungen ermittelt:

* MRS-Medium setzt sich wie folgt zusammen:
pro 1:
2 g Fleischextrakt/MERCK - 10 g Pepton aus Casein, trypisch - 4 g Hefe-Extrakt - 20 g Glucose - 1 ml Tween 80 - 2,5 g $K_2HPO_4$ x 3 $H_2O$ - 5 g Na-Acetat x $3H_2O$ - 2 g Di-Ammoniumcitrat - 200 mg $MgSO_4$ x 7 $H_2O$ - 50 mg $MnSO_4$ x $H_2O$.

(1) Pyruvat + $P_i$ + $O_2$ $\xrightarrow{\text{Py-OD}}$ Acetyl-P + $CO_2$ + $H_2O_2$

(2) $H_2O_2$ + 4-Aminoantipyrin(4-AA) + 2-Hydroxy-3,5-dichlorbenzolsulfonsäure(HDBS) $\xrightarrow{\text{POD}}$ Chinon-Farbstoff (546 nm) + $2H_2O$

$H_2O_2$ wird in äquimolarer Menge des Farbstoffes verbraucht.
1 Unit Py-OD = 1 µMol Pyruvat-Umsatz/min bei 25 °C.

Bestimmung von Pyruvat:

72 mM Kaliumphosphatpuffer, pH 6,7; 8 mM 4-AA; 6,8 mmol/l HDBS; 0,01 mmol/l FAD; 2 U POD; 10 U Py-OD pro ml. Der Start für einen Endwerts-Ablauf erfolgt durch Zugabe von 0,05 µMol Pyruvat bzw. Pyruvat enthaltende Probe in ein Küvetten-Volumen von 2 ml bei 1 cm Schichtdicke und 37 °C.

Die Reaktion kommt nach 15 Minuten zum Stillstand. Ein Extinktionskoeffizient bei 546 nm von $\epsilon$ = 16,5 $cm^2$/µMol wurde für reinstes Pyruvat (Mononatriumsalz) gemessen.

**Beispiel 2**

Kinetische Bestimmung der GPT

Zu 2,5 ml einer Lösung enthaltend:

| | |
|---|---|
| $KH_2PO_4$ | 80 mmol/l pH 6,7 |
| Alanin | 800 mmol/l |

N-(3'-Sulfonyl-4'-hydroxy-5'-chlorphenyl)-4-aminoantipyrin, 0,2 mmol/l α-Ketoglutarsäure 18 mmol/l

| | | |
|---|---|---|
| Pyruvatoxidase, | 0,2 | U/ml |
| POD | 2 | U/ml |

werden
Probe 0,1 ml
gegeben. Nach 3 Minuten wird die Extinktionsänderung pro Minute bestimmt. Die Meßtemperatur ist 25°, die Wellenlänge 546 nm. Das $\epsilon$ beträgt 19,6, das Gesamtvolumen 2,6 ml und das Probevolumen 0,1 ml. Aus der Extinktionszunahme pro Minute errechnet sich die Aktivität an GPT.

**Beispiel 3**

Kinetische Bestimmung der GOT

In einem Ansatz, analog Beispiel 2, der anstelle von Alanin 200 mmol/l Alaninsulfinsäure enthält, wird unter den gleichen Bedingungen wie in Beispiel 2 eine GOT-haltige Probe zugefügt. Aus der Extinktionsänderung pro Minute wird der Gehalt an GOT direkt berechnet.

### Beispiel 4

Teststreifen für GPT

Zur Herstellung von Teststreifen von GPT werden 2 Papiere imprägniert:

A) Enzympapier

| | |
|---|---|
| 0,5 Mol/l | Morpholinethansulfonsäure/Kaliumhydroxyd, pH 6,5 (MOPS-Puffer) |
| 800 mmol/l | Alanin |
| 1 mmol/l | Kaliumhydrogenphosphat |
| 20000 U/l | POD |
| 200000 U/l | Pyruvatoxidase |

Mit dieser Lösung wird ein saugfähiges Papier, Dicke 50 µm, Flächengewicht 12 g/m$^2$, Saugfähigkeit 50 g $H_2O/m^2$, imprägniert und 5 Minuten bei 30° getrocknet (z. B. Teebeutelpapier der Firma Schöller & Hösch). Das Papier wird anschließend auf 1 cm Breite geschnitten.

B) Indikatorpapier

| | |
|---|---|
| 10 mmol/l | 4,5-(4-Dimethylamino-phenyl-)-2-(3,5-Dimethoxy-4-hydroxyphenyl)-imidazol |
| 18 mmol/l | α-Ketoglutarsäure gelöst in |
| 100 mmol/l | HCl (Puffersystem). |

Mit dieser Lösung wird ebenfalls ein entsprechend saugfähiges Papier imprägniert, 5 Minuten bei 30° getrocknet und auf 1 cm Breite geschnitten. Das Material wird verarbeitet, indem eine 1 cm breite, durchsichtige Polycarbonatfolie von 100 µm Dicke, gemeinsam mit dem Indikatorpapier und dem Enzympapier einseitig auf einem Plastikstreifen befestigt wird, so daß die Folie nach außen, das Indikatorpapier in die Mitte, das Enzympapier nach innen zu liegen kommt. Daneben wird ein 15 mm breites Glasfaservlies, Dicke 1,5 mm, Faserdicke ca. 2 µm aufgebracht, so daß das freie Ende der Folie und der getränkten Papiere noch 6 mm über das Vlies reichen. Dann wird in 6 mm breite Teststreifen geschnitten. Bringt man nun 15µl Vollblut auf den Probenauftragsbezirk, so durchdringt innerhalb von 30 bis 60 Sekunden der Plasmaanteil das gesamte Glasfaservlies auch unterhalb der durchsichtigen Folie, während die Erythrozyten an der Auftragstelle festgehalten werden. Nach Andrücken der Folie kommen nun das Enzym- und das Indikatorpapier mit dem entwickelten Plasma in Berührung und werden gleichmäßig durchfeuchtet. Die im Plasma enthaltene GPT reagiert unter Blaufärbung, deren Intensität proportional zur GPT-Menge ist und gegebenenfalls im Remissionsphotometer gemessen werden kann. In gleicher Weise reagieren auch die anderen Probenmaterialien wie Serum, Plasma, etc.

### Beispiel 5

Teststreifen auf GOT

Die Teststreifen auf GOT werden in vollständig gleicher Weise zubereitet wie die Streifen auf GPT gemäß Beispiel 4, nur daß in dem Enzympapier anstelle von Alanin 200 mmol Alaninsulfinsäure enthalten ist. Hier ist die nach dem Andrücken der transparenten Folie gebildete blaue Farbe ein Maß für die Menge an GOT in der Probe.

## Ansprüche

**1.** Pyruvatoxidase, welche ohne Zusatz von FAD, TPP und zweiwertigen Metallionen Pyruvat unter Bildung von $H_2O_2$ decarboxyliert, erhältlich aus Lactobacillus plantarum, DSM 2571, Lactobacillus salivarius, DSM 2573, Leuconostoc mesenteroides, DSM 2572 oder Streptococcus cremoris, DSM 2574.

**2.** Pyruvatoxidase nach Anspruch 1,

**gekennzeichnet** durch ein Aktivitätsoptimum bei pH 5,7, ein Stabilitätsoptimum bei pH 5,6 bis 5,8, eine $K_M$ für Pyruvat bei 25 °C von 0,4 mmol/l, eine $K_M$ für Phosphat bei 25 °C von 2,3 mmol/l und ein Molekulargewicht von 146.000 Dalton bei Bestimmung in der Ultrazentrifuge nach Ames.

**3.** Verfahren zur Herstellung der Pyruvatoxidase gemäß Anspruch 1 oder 2, **dadurch gekennzeichnet,** daß man einen auf einem pyruvathaltigen Medium gezüchteten, Pyruvatoxidase enthaltenden Mikroorganismus aufschließt, den wäßrigen Aufschluß mit Polyethylenimin versetzt, die unlöslichen Anteile abtrennt und aus der erhaltenen Lösung Pyruvatoxidase durch Gelchromatographie und Fraktionierung mit Ammoniumsulfat isoliert.

**4.** Verfahren nach Anspruch 3, **dadurch gekennzeichnet**, daß man als Mikroorganismen Milchsäurebakterien verwendet.

**5.** Verfahren nach Anspruch 4, **dadurch gekennzeichnet,** daß man als Milchsäurebakterien Lactobacillus plantarum DSM 2571, Lactobacillus salivarius DSM 2573, Leuconostoc mesenteroides DSM 2572 oder/und Streptococcus cremoris DSM 2574 verwendet.

**6.** Verwendung des Enzyms von Anspruch 1 oder 2 zur Bestimmung von Pyruvat durch Messung von gebildetem $H_2O_2$ oder verbrauchtem $O_2$.

**7.** Reagenz zur Bestimmung von Pyruvat, **dadurch gekennzeichnet,** daß es Pyruvatoxidase nach Anspruch 1 oder 2, Phosphat, Puffer und ein System zur Bestimmung von $H_2O_2$ enthält.

**8.** Reagenz nach Anspruch 7, **dadurch gekennzeichnet,** daß es auf einem blattförmigen Trägermaterial inkorporiert vorliegt.

**9.** Reagenz nach Anspruch 8, **dadurch gekennzeichnet,** daß das System zur Bestimmung von $H_2O_2$ aus Peroxidase und als Indikator
a) 4-Aminoantipyrin und 2-Hydroxy-3,5-dichlorbenzol-sulfonsäure oder
b) 4,5-(4-Dimethylaminophenyl)-2-(3,5-dimethoxy-4-hydroxyphenyl)-imidazol besteht.

**10.** Reagenz nach Anspruch 8 oder 9, **dadurch gekennzeichnet,** daß es aus einem Trägerblattsandwich besteht und ein Trägerblatt den Indikator und ein weiteres Trägerblatt die übrigen Bestandteile enthält.

Patentanspruch für folgenden Vertragsstaat: AT

**1.** Verfahren zur Herstellung einer Pyruvatoxidase, welche ohne Zusatz von FAD, TPP und zweiwertigen Metallionen Pyruvat unter Bildung von $H_2O_2$ decarboxyliert, **dadurch gekennzeichnet,** daß man einen auf einem pyruvathaltigen Medium gezüchteten, Pyruvatoxidase enthaltenden Mikroorganismus, insbesondere Lactobacillus plantarum, DSM 2571, Lactobacillus salivarius, DSM 2573, Leuconostoc mesenteroides, DSM 2572 oder Streptococcus cremoris, DSM 2574, aufschließt, den wäßrigen Aufschluß mit Polyethylenimin versetzt, die unlöslichen Anteile abtrennt und aus der erhaltenen Lösung Pyruvatoxidase durch Gelchromatographie und Fraktionierung mit Ammoniumsulfat isoliert.

## Claims

**1.** Pyruvate oxidase which, without addition of FAD, TPP and divalent metal ions, decarboxylates pyruvate with the formation of $H_2O_2$, obtainable from Lactobacillus plantarum, DSM 2571, Lactobacillus salivarius, DSM 2573, Leuconostoc mesenteroides, DSM 2572 or Streptococcus cremoris, DSM 2574.

**2.** Pyruvate oxidase according to claim 1, characterised by an activity optimum at pH 5.7, a stability optimum at pH 5.6 to 5.8, a $K_M$ for pyruvate at 25 °C. of 0.4 mmol/l., a $K_M$ for phosphate at 25 °C. of 2.3 mmol/l. and a molecular weight of 146,000 Dalton in the case of determination in an ultracentrifuge according to Ames.

**3.** Process for the preparation of the pyruvate oxidase according to claim 1 or 2, characterised in that one digests a micro-organism cultured on a pyruvate-containing medium and containing pyruvate oxidase,

mixes the aqueous digest with polyethyleneimine, separates off the insoluble parts and isolates pyruvate oxidase from the solution obtained by gel chromatography and fractionation with ammonium sulphate.

**4.** Process according to claim 3, characterised in that one uses lactic acid bacteria as micro-organisms.

**5.** Process according to claim 4, characterised in that as lactic acid bacteria one uses Lactobacillus plantarum DSM 2571, Lactobacillus salivarius DSM 2573, Leuconostoc mesenteroides DSM 2572 and/or Streptococcus cremoris DSM 2574.

**6.** Use of the enzyme of claim 1 or 2 for the determination of pyruvate by measurement of $H_2O_2$ formed or of consumed $O_2$.

**7.** Reagent for the determination of pyruvate, characterised in that it contains pyruvate oxidase according to claim 1 or 2, phosphate, buffer and a system for the determination of $H_2O_2$.

**8.** Reagent according to claim 7, characterised in that it is present on a leaf-shaped carrier material.

**9.** Reagent according to claim 8, characterised in that the system for the determination of $H_2O_2$ consists of peroxidase and, as indicator
a) 4-aminoantipyrine and 2-hydroxy-3,5-dichloro-benzene-sulphonic acid or
b) 4,5-(4-dimethylaminophenyl)-2-(3,5-dimethoxy-4-hydroxyphenyl)-imidazole.

**10.** Reagent according to claim 8 or 9, characterised in that it consists of a carrier leaf sandwich and one carrier leaf contains the indicator and a further carrier leaf the other components.

Claim for the Contracting State: AT

**1.** Process for the preparation of a pyruvate oxidase which, without addition of FAD, TPP and divalent metal ions, decarboxylates pyruvate with formation of $H_2O_2$, characterised in that one digests a micro-organism cultured on a pyruvate-containing medium and containing pyruvate oxidase, especially Lactobacillus plantarum, DSM 2571, Lactobacillus salivarius, DSM 2573, Leuconostoc mesenteroides, DSM 2572 or Streptococcus cremoris, DSM 2574, mixes the aqueous digest with polyethyleneimine, separates off the insoluble parts and isolates pyruvate oxidase from the solution obtained by gel chromatography and fractionation with ammonium sulphate.

## Revendications

**1.** Pyruvate oxydase qui, sans addition de FAD, de TPP et d'ions métalliques bivalents, décarboxyle le pyruvate avec formation de $H_2O_2$, pyruvate oxydase qui peut être obtenue à partir de Lactobacillus plantarum, DSM 2571, Lactobacillus salivarius, DSM 2573, Leuconostoc mesenteroides, DSM 2572 ou Streptococcus cremoris, DSM 2574.

**2.** Pyruvate oxydase selon la revendication 1, caractérisée par un optimum d'activité à pH 5,7, un optimum de stabilité à pH 5,6 à 5,8, une $K_M$ pour le pyruvate à 25 °C de 0,4 mmol/l, une $K_M$ pour le phosphate à 25 °C de 2,3 mmol/l et un poids moléculaire de 146.000 daltons par détermination à l'ultracentrifugeuse selon Ames.

**3.** Procédé de préparation de la pyruvate oxydase selon la revendication 1 ou 2, caractérisé en ce qu'on désagrége un micro-organisme contenant de la pyruvate oxydase, cultivé sur un milieu contenant du pyruvate, on ajoute de la polyéthylèneimine au produit de désagrégation aqueux, on sépare les constituants insolubles et on isole la pyruvate oxydase à partir de la solution obtenue par chromatographie sur gel et fractionnement au sulfate d'ammonium.

**4.** Procédé selon la revendication 3, caractérisé en ce qu'on utilise comme micro-organismes des bactéries lactiques.

**5.** Procédé selon la revendication 4, caractérisé en ce qu'on utilise comme bactéries lactiques Lactobacillus plantarum DSM 2571, Lactobacillus salivarius DSM 2573, Leuconostoc mesenteroides DSM 2572 ou/et Streptococcus cremoris DSM 2574.

**6.** Utilisation de l'enzyme selon la revendication 1 ou 2 pour la détermination du pyruvate par mesure du $H_2O_2$ formé ou du $O_2$ consommé.

**7.** Réactif pour la détermination du pyruvate, caractérisé en ce qu'il contient de la pyruvate oxydase selon la revendication 1 ou 2, du phosphate, du tampon et un système pour la détermination de $H_2O_2$.

**8.** Réactif selon la revendication 7, caractérisé en ce qu'il se présente incorporé sur un matériau support sous forme de feuille.

**9.** Réactif selon la revendication 8, caractérisé en ce que le système pour la détermination de $H_2O_2$ est constitué de peroxydase et comme indicateur de
a) 4-aminoantipyrine et acide 2-hydroxy-3,5-dichloro-benzène-sulfonique ou
b) 4,5-(4-diméthylaminophényl)-2-(3,5-diméthoxy-4-hydroxyphényl)-imidazole.

**10.** Réactif selon la revendication 8 ou 9, caractérisé en ce qu'il est constitué d'un sandwich de feuilles supports et en ce qu'une feuille support contient l'indicateur et une autre feuille support contient les autres constituants.

REVENDICATION POUR L'ETAT CONTRACTANT: AT

**1.** Procédé de préparation d'une pyruvate oxydase qui, sans addition de FAD, de TPP et d'ions métalliques bivalents, décarboxyle le pyruvate avec formation de $H_2O_2$, caractérisé en ce qu'on désagrège un micro-organisme contenant de la pyruvate oxydase, cultivé sur un milieu contenant du pyruvate, en particulier Lactobacillus plantarum, DSM 2571, Lactobacillus salivarius, DSM 2573, Leuconostoc mesenteroides, DSM 2572 ou Streptococcus cremoris, DSM 2574, on ajoute de la polyéthylènimine au produit de désagrégation aqueux, on sépare les constituants insolubles et on isole la pyruvate oxydase à partir de la solution obtenue par chromoatographie sur gel et fractionnement au sulfate d'ammonium.